(19) Europäisches Patentamt
European Patent Office
Office européen des brevets

(11) **EP 1 997 901 B1**

(12) **EUROPÄISCHE PATENTSCHRIFT**

(45) Veröffentlichungstag und Bekanntmachung des Hinweises auf die Patenterteilung:
**19.01.2011 Patentblatt 2011/03**

(51) Int Cl.:
*C12P 5/02* *(2006.01)*

(21) Anmeldenummer: **08004314.4**

(22) Anmeldetag: **08.03.2008**

(54) **Verfahren zur Erzeugung von Biogas**

Process for producing biogas

Procédé de production de biogaz

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **29.05.2007 DE 102007025155**

(43) Veröffentlichungstag der Anmeldung:
**03.12.2008 Patentblatt 2008/49**

(73) Patentinhaber: **IS Forschungsgesellschaft mbH
25421 Pinneberg (DE)**

(72) Erfinder:
• **Oechsner, Hans
72644 Oberboihingen (DE)**
• **Lemmer, Andreas
70599 Stuttgart (DE)**
• **Ramhold, Dietmar
23820 Strenglin (DE)**
• **Mathies, Edmund
25436 Moorrege (DE)**
• **Mayrhuber, Elisabeth
8605 Kapfenberg (AT)**
• **Preißler, Daniel
70599 Stuttgart (DE)**

(74) Vertreter: **Siemons, Norbert
Hauck Patent- und Rechtsanwälte
Neuer Wall 50
20354 Hamburg (DE)**

(56) Entgegenhaltungen:
**SE-C2- 529 177**

• **DATABASE COMPENDEX [Online] ENGINEERING INFORMATION, INC.; WATE, S.R. ET AL.: "Effect of cobalt on biogas production from cattle dung" XP002501570 Database accession no. EIX84060098543 & INDIAN JOURNAL OF ENVIRONMENTAL HEALTH 1983 JUL IN, Bd. 25, Nr. 3, Juli 1983 (1983-07), Seiten 179-190,**
• **ARESTA, M. ET AL.: "INFLUENCE OF IRON, NICKEL AND COBALT ON BIOGAS PRODUCTION DURING THE ANAEROBIC FERMENTATION OF FRESH RESIDUAL BIOMASS" CHEMISTRY AND ECOLOGY, Bd. 19, Nr. 6, Dezember 2003 (2003-12), Seiten 451-459, XP008097934**
• **WILKIE, A. ET AL.: "Enhancement of Ananerobic Methanogenesis from Napiergrass by Addition of Micronutrients" BIOMASS, Bd. 11, 1986, Seiten 135-146, XP002501569**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung bezieht sich auf ein Verfahren zur Biogaserzeugung aus organischer Masse in einem Biogasreaktor (nachfolgend auch Fermenter genannt).

Die Fixierung von Sonnenenergie in Biomasse durch die pflanzliche Photosynthese ist eine der bedeutendsten sich selbst erneuernden Energiequellen (Maurer, M. und Winkler, J.-P; Biogas. Theoretische Grundlagen, Bau und Betrieb von Anlagen; 1982; Springer-Verlag). Aufbauend auf der Energiegewinnung durch die Photosynthese werden als Stoffwechselleistung von den Pflanzen Makromoleküle synthetisiert. Diese Makromoleküle können beim anaeroben Abbau in Biogasanlagen mit einer sehr hohen Effizienz in Methan und Kohlendioxid umgewandelt werden, so dass bis zu 82 % der in den Pflanzen gespeicherten Energie in Methan überführt werden.

Der Prozess der Biogaserzeugung kann in vier Stufen unterteilt werden. In einem ersten Schritt, der Hydrolyse, werden die komplexen Strukturen der Biomasse in ihre Monomere (Zucker, Fette, Eiweiße) zerlegt. Anschließend erfolgt ein Abbau der Monomere zu kurzkettigen Fettsäuren (Acidogenese). Im dritten (Acetogenese) und vierten Schritt (Methanogenese) erfolgt zunächst die Bildung von Essigsäure und in der Folge von Methan. Als Nebenprodukte entstehen im Biogasprozess vor allem Kohlendioxid und weitere Gase in geringen Konzentrationen. Die optimalen Milieubedingungen unterscheiden sich in den jeweiligen Schritten zum Teil erheblich. (SAHM: Biologie der Methanbildung, Chem.-Ing. Tech.53 (1981) Nr. 11, S. 854 - 863).

Gemäß dem Stand der Technik erfolgt der anaerobe Abbau organischer Substanz im wässrigen Milieu mit Trockensubstanzgehalten von in der Regel unter 30 %.

Die Erzeugung von Biogas erfolgt in Abhängigkeit der am Prozess beteiligten Mikroorganismen bei verschiedenen Temperaturoptima im Bereich von 25-57 °C.

Das optimale Kohlenstoff:Stickstoff:Phosphor:Schwefel-Verhältnis liegt bei 500:15:5:3 für die Hydrolyse und Acidogenese, bzw. 600:15:5:3 für die Acetogenese und Methanogense.

Der für die Hydrolyse und Acidogenese optimale pH-Wert liegt im Bereich von pH 5,2-6,3, der für die Acetogenese und Methanogense optimale pH-Wert im Bereich von pH 6,7-7,5.

Als Gärsubstrate kommen feste und flüssige Substrate zum Einsatz. Sowohl biogene Abfälle aus Industrie, Gewerbe, Landwirtschaft und Haushalten als auch gezielt zur Erzeugung von Methan angebaute Energiepflanzen werden in Biogasanlagen eingesetzt. Häufig werden in landwirtschaftlichen Biogasanlagen zusätzlich tierische Exkremente dem Prozess zugeführt, um deren Energiepotenzial zusätzlich auszuschöpfen. Vielfach wird dem Biogasreaktor zu Beginn des Prozesses der Biogaserzeugung Gülle zusammen mit den geernteten Energiepflanzen zugeführt und wird der Biogasreaktor danach ausschließlich mit den geernteten Energiepflanzen gefüttert. Die Erfindung bezieht sich auf sämtliche Varianten der Biogaserzeugung.

Der letzte der Abbauschritte, die Methanbildung, erfolgt durch die methanogenen Mikroorganismen, die zur Gruppe der Archaea (Archacbakterien) gehören. Zusammen mit den Halobakterien und einigen Hyperthermophilen Gärern bilden sie den Zweig der Euryarcheota (-Schlegel, H.-G.; Allgemeine Mikrobiologie; 8. Aufl., 2007, Georg Thieme Verlag). Unter allen Lebewesen nehmen die Methanogenen eine Sonderstellung ein. Viele ihrer Stoffwechselprozesse können nur mit Hilfe von Coenzymen ablaufen, die bei anderen Mikroorganismen nur sehr vereinzelt eine Rolle spielen. Eines der bisher 7 bekannten ist das Coenzym F430, ein Cofaktor mit einem Nickel-Zentralion. Ein weiteres Beispiel ist die Formyl-Mehthanofuran-Dehydrogenase mit einem Molybdän-Cofaktor (SCHLEGEL, a.a.O. 2007). Aufgrund dieser einzigartigen Stoffwechselprozesse stellen die methanogenen Organismen besondere Ansprüche an die Konzentration von Spurenelementen.

Bereits bekannt ist, dem Fermenter von Biogasanlagen Zusätze zuzuführen, die Spurenelemente enthalten. Die EP 1 577 269 A1 offenbart einen Zusatz eines mit Spurenelementen beladenen Zeolith zum Ausgleich eines Mangels an für die Methangasbakterien wichtigen Spurenelementen. Das Gärsubstrat ist beispielsweise eine Mischung aus Schweinegülle und Maissilage. Bei Zugabe bekannter Zusätze mit Spurenelementen werden teilweise nur zeitweilige, geringe oder überhaupt keine Verbesserungen der Biogaserzeugung erreicht.

Die SE 529 177 C2 beschreibt ein Verfahren zur Biogaserzeugung aus Biomasse in einem Biogasreaktor. Bei dem Verfahren wird ein Richtwert für die Konzentration von Eisen und Kobalt und Salzsäure in einem Biogasreaktor für eine effektive Biogaserzeugung bereitgestellt. In dem Biogasreaktor wird aus Biomasse Biogas erzeugt. Die Konzentration von Eisen und/oder kobalt und/oder pH-Wert in der Biomasse im Biogasreaktor wird ermittelt und im Falle einer Unterschreitung des Richtwertes durch die ermittelten konzentrationen fehlende Spurenelemente und Salzsäure dem Biogasreaktor zugegeben.

Aus DATABASE COMPENDEX [Online], ENGINEERING INFORMATION; INC.; WATE, S.R. ET AL.: "Effect of cobalt on biogas production from cattle dung" Database accession no. EIX84060098543 ist ein Verfahren zur Biogaserzeugung aus Biomasse in einem Biogasreaktor bekannt, bei dem durch Kobalt-Zugabe auf eine Gesamtkonzentration von 310 mg/kg TS eine 20 %ige Erhöhung der Biogasproduktion erreicht wird.

ARESTA, M. ET AL.: "INFLUENCE OF IRON; NICKEL AND COBALT ON BIOGAS PRODUCTION DURING THE ANAEROBIC FERMENTATION OF FRESH RESIDUAL BIOMASS"; CHEMISTRY AND ECOLOGY, Bd. 19, Nr.

6, Dezember 2003, Seiten 451-459 und WILKY, A. ET AL.: "Enhancement of Ananerobic Methanogenesis from Napiergrass by Addition of Micronutrients", BIOMASS, Bd. 11, 1986, Seiten 135-146 offenbaren Verfahren zur Biogaserzeugung aus Biomasse in einem Biogasreaktor, bei dem im erstgenannten Dokument durch Zugabe von Nickel, Kobalt und Eisen und im zweitgenannten Dokument von Nickel, Kobalt und Molybdän eine Erhöhung der Biogasproduktion erreicht wird.

Davon ausgehend liegt der Erfindung die Aufgabe zugrunde, ein weiteres Verfahren zur Biogaserzeugung zur Verfügung zu stellen, das eine verbesserte Versorgung der Mikroorganismen mit Spurenelementen aufweist.

Die Aufgabe wird durch ein Verfahren mit den Merkmalen des Anspruches 1 gelöst. Vorteilhafte Ausgestaltungen des Verfahrens sind in den Unteransprüchen angegeben.

Das crfindungsgemäße Verfahren zur Biogaserzeugung aus Biomasse in einem Biogasreaktor umfaßt die folgenden Schritte:

- mindestens ein Richtwert für die Konzentration mindestens eines Spurenelementes in einem Biogasreaktor für eine effektive Biogaserzeugung wird bereitgestellt,
- in dem Biogasreaktor wird aus Biomasse Biogas erzeugt,
- die Konzentration mindestens eines Spurenelementes in der Biomasse im Biogasreaktor wird ermittelt,
- im Falle einer Unterschreitung des Richtwertes eines Spurenelementes durch die ermittelte Konzentration des Spurenelementes wird dieses Spurenelement dem Biogasreaktor zugegeben,
- wobei die Richtwerte für Nickel 4 bis 30 mg/kg TS und/oder für Kobalt 0,4 bis 5 mg/kg TS und/oder für Molybdän 1,0 bis 10,0 mg/kg TS und/oder für Eisen 1500 bis 3500 mg/kg TS betragen.

Die Erfindung geht von der überraschenden Erkenntnis aus, daß die Biogaserzeugung im Biogasreaktor besonders effektiv ist, wenn die Konzentration mindestens eines für die Biogaserzeugung maßgeblichen Spurenelementes einen Richtwert einhält. Maßgebliche Spurenelemente und Richtwerte für ihre Konzentration im Biogasreaktor wurden durch Untersuchungen mit Laboranlagen und Praxisanlagen ermittelt. Es ist anzunchmen, daß durch weitere Untersuchungen weitere Erkenntnisse gewonnen werden können, die die Bereitstellung weiterer bzw. genauerer Richtwerte ermöglicht. Bei dem erfindungsgemäßen Verfahren wird die tatsächliche Konzentration mindestens eines Spurenelementes in der Biomasse im Biogasreaktor (auch "Fermenterinhalt" oder "Gärsubstrat" genannt) ermittelt. Bei der Biomasse handelt es sich insbesondere um die eingangs erwähnten Gärsubstrate gegebenenfalls zuzüglich darin enthaltener oder zugesetzter Mikroorganismen. Unterschreitet die Konzentration den Richtwert, so wird das betreffende Spurenelement dem Biogasreaktor zugegeben. Dabei kann die Zugabe des Spurenelementes auf Fälle beschränkt werden, in denen eine deutliche Unterschreitung des Richtwertes (z.B. um eine vorgegebene Toleranz) gegeben ist. Überschreitet die tatsächliche Konzentration des Spurenelementes den Richtwert (ggfs. abzüglich der Toleranz), so wird von einer Zugabe des Spurenelementes abgesehen. Überhöhte Konzentrationen der Spurenelemente sollten nämlich vermieden werden, da die Biogaserzeugung im Biogasreaktor hierdurch geschädigt werden kann. Außerdem führen Überdosierungen dazu, daß die Flächen, auf die Gärrückstände ausgebracht werden, unnötig mit Schwermetallen belastet werden. Durch Einhalten des Richtwertes mindestens eines Spurenelementes wird somit eine effektivere Biogaserzeugung erreicht. Bevorzugt wird für mehrere Spurenelemente die Einhaltung der Richtwerte kontrolliert und gegebenenfalls durch Zugabe von Spurenelementen sichergestellt. Die Spurenelementzugabe dient damit der Stabilisierung und Leistungssteigerung der Methangasbildung aus organischer Substanz. Wird ein Spurenelementmangel im Gärsubstrat ausgeglichen, so wird die Leistungsfähigkeit und die Populationsdichte der im Fermenter enthaltenen Biologie gesteigert und somit eine Erhöhung des Substratdurchsatzes in der Biogasanlage ermöglicht.

Die Untersuchungen haben gezeigt, daß die Kontrolle der Einhaltung von Richtwerten bestimmter Spurenelemente für die Wirksamkeit der Biogaserzeugung besonders bedeutsam ist. Es handelt sich hierbei um die Spurenelemente Nickel, Kobalt, Molybdän und Eisen. Deshalb werden gemäß einer Ausgestaltung des Verfahrens Richtwerte für die Konzentrationen der Spurenelemente Nickel und/oder Kobalt und/oder Molybdän und/oder Eisen bereitgestellt und die Konzentrationen der Spurenelemente Nickel und/oder Kobalt und/oder Molybdän und/oder Eisen in der Biomasse im Biogasreaktor ermittelt. Ein eventueller Mangel der genannten Spurenelemente im Biogasreaktor kann dann ausgeglichen werden.

Die Richtwerte betragen für Nickel 4 bis 30 mg/kg TS und/oder für Kobalt 0,4 bis 5 mg/kg TS und/oder für Molybdän 1,0 bis 10,0 mg/kg TS und/oder für Eisen 1500 bis 3500 mg/kg TS.

Gemäß einer weiteren Ausgestaltung betragen die Richtwerte für Nickel mindestens 10 und/oder maximal 25 mg/kg TS und/oder für Kobalt mindestens 1,0 mg/kg TS.

Nach den derzeitigen Erkenntnissen betragen die optimalen Richtwerte für Nickel 16 mg/kg TS und/oder für Kobalt 1,8 mg/kg TS und/oder für Molybdän 4 mg/kg TS und/oder für Eisen 2400 mg/kg TS.

Ferner haben die Untersuchungen ergeben, daß weitere Spurenelemente bei der Biogaserzeugung bedeutsam sind. Hierbei handelt es sich um die Spurenelemente Mangan, Kupfer, Selen, Wolfram und Zink. Gemäß einer Ausgestaltung des Verfahrens werden deshalb Richtwerte für die Konzentration der Spurenelemente Mangan und/oder Kupfer und/oder Selen und/oder Wolfram und/oder Zink bereitgestellt und die Konzentrationen der Spurenelemente Mangan und/oder Kupfer und/oder Selen und/oder Wolfram und/oder Zink im Biogasreaktor ermittelt. Im Falle eines Mangels wird das betreffende Spurenelement dem Biogasreaktor zugesetzt.

Gemäß einer weiteren Ausgestaltung betragen die Richtwerte für Mangan 100 bis 1500 mg/kg TS und/oder für Kupfer 10 bis 80 mg/kg TS und/oder für Selen 0,05 bis 4 mg/kg TS und/oder für Wolfram 0,1 bis 30 mg/kg TS und/oder für Zink 30 bis 400 mg/kg TS.

Gemäß einer weiteren Ausgestaltung betragen die Richtwerte für Mangan mindestens 250 und/oder maximal 350 mg/kg TS und/oder für Kupfer mindestens 30 und/oder maximal 50 mg/kg TS und/oder für Selen mindestens 0,3 und/oder maximal 0,7 mg/kg TS und/oder für Wolfram mindestens 0,4 und/oder maximal 0,8 mg/kg TS und/oder für Zink mindestens 150 und/oder maximal 250 mg/kg TS.

Nach den derzeitigen Erkenntnissen betragen die optimalen Konzentrationen für Mangan 300 mg/kg TS und/oder für Kupfer 40 mg/kg TS und/oder für Selen 0,5 mg/kg TS und/oder für Wolfram 0,6 mg/kg TS und/oder für Zink 200 mg/kg TS.

Ein Ausgleich eines Mangels an Spurenelementen sollte unter Berücksichtigung der biologischen Verfügbarkeit und des tatsächlichen Bedarfs erfolgen. Gemäß einer Ausgestaltung des Verfahrens wird zunächst die Verfügbarkeit der bereits im Gärsubstrat enthaltenen Spurenelemente erhöht. Dies kann z.B. durch Änderung physikalischer Parameter des Verfahrens wie Temperatur, Druck, Trockensubstanzanteil, Wassergehalt, Mischintensität erfolgen. Gemäß einer Ausgestaltung wird dem Biogasreaktor ein die biologische Verfügbarkeit der Spurenelemente erhöhender Zusatz zugeführt. Die biologische Verfügbarkeit der Spurenelemente wird durch hohe Sulfid-Konzentration herabgesetzt; schwer lösliche und nicht biologisch verfügbare Metallsulfide fallen aus. Gemäß einer Ausgestaltung des Verfahrens wird die biologische Verfügbarkeit durch Zusatz eines die Sulfid-Konzentration herabsetzenden Mittels erhöht. Aufgrund der guten Affinität von Eisen zu Sulfid können durch Eisenzugabe die Sulfid-Ionen gebunden werden, so daß nur in geringen Mengen zugeführte Spurenelemente in einem geringeren Umfang durch die Sulfide festgelegt werden. Positiv wirkt sich dabei aus, daß Eisen selbst in hohen Konzentrationen nicht zu einer Hemmung der Biogaserzeugung im Fermenter führt. Deshalb wird gemäß einer Ausgestaltung des Verfahrens das Spurenelement Eisen dem Biogasreaktor zugegeben.

Gemäß einer weiteren Ausgestaltung des Verfahrens wird zunächst die Verfügbarkeit der bereits im Gärsubstrat enthaltenen Spurenelemente erhöht und danach ein Mangel durch Zusatz von Spurenelementen ausgeglichen. Hierdurch wird eine direkte Verminderung der biologischen Verfügbarkeit der zum Mangelausgleich zugegebenen Spurenelemente - z.B. durch Festlegung an den Sulfiden - vermieden.

Gemäß einer weiteren Ausgestaltung des Verfahrens wird nach dem Erhöhen der biologischen Verfügbarkeit der Spurenelemente die Konzentration mindestens eines Spurenelements in der Biomasse ermittelt und ein Mangel des Spurenelementes durch Zugabe desselben kompensiert. Hierdurch wird eine bessere Ausnutzung der im Gärsubstrat enthaltenen Spurenelemente und die Einstellung optimaler Konzentrationen der Spurenelemente in der Biomasse begünstigt.

Die Konzentration des mindestens einen Spurenelementes im Biogasreaktor kann auf verschiedene Weise ermittelt werden. Gemäß einer Ausgestaltung des Verfahrens wird die Konzentration durch ICP(inductive coupled plasma)-Analyse mindestens einer Probe aus dem Biogasreaktor ermittelt.

Grundsätzlich braucht die Konzentration des mindestens einen Spurenelementes nur einmal ermittelt zu werden, um die Einhaltung des zugeordneten Richtwertes zu überprüfen und gegebenenfalls das betreffende Spurenelement zuzugeben. Die Spurenelementekonzentrationen innerhalb des Fermenters sind von den jeweils zugeführten Substraten abhängig und können sich deshalb mit der Fütterung des Fermenters ändern. Ferner kann die biologische Verfügbarkeit der Spurenelemente durch die zugesetzten Substrate und Prozesshilfsstoffe beeinflußt werden und kann sich deshalb mit der Zeit ändern. Zur Erfassung von Änderungen der Konzentrationen der Spurenelemente im Biogasreaktor wird gemäß einer Ausgestaltung des Verfahrens die Konzentration mindestens eines Spurenelementes im Biogasreaktor in Zeitabständen wiederholt ermittelt. Die jeweils aktuelle Konzentration des mindestens einen Spurenelementes wird mit dem zugeordneten Richtwert verglichen und einer aktuellen Berechnung der Zugabe zugrunde gelegt.

Die zuzugebende Menge der Spurenelemente kann auf verschiedene Weise ermittelt werden. Beispielsweise kann im Falle eines Mangels eines Spurenelementes eine vorgegebene Menge des Spurenelementes einmalig oder in Zeitabständen mehrfach zugegeben werden. In einem Zeitabstand kann die Konzentration des Spurenelementes im Biogasreaktor ermittelt werden. Aufgrund der ermittelten Konzentration kann festgestellt werden, ob eine erneute Zugabe der vorgegebenen oder einer abweichenden Menge erforderlich ist. Wird der Richtwert immer noch unterschritten, kann die vorgegebene Zugabe entsprechend dem Verhältnis des Richtwertes zur gemessenen aktuellen Konzentration gesteigert werden. Wird der Richtwert überschritten, kann die vorgegebene Zugabe entsprechend dem Verhältnis des Richtwertes zur gemessenen aktuellen Konzentration reduziert werden. Auf diese Weise ist eine Optimierung der zuzugebenden Menge möglich.

Gemäß einer anderen Ausgestaltung wird nicht anfänglich eine vorgegebene Menge des Spurenelementes zugegeben. Vielmehr wird die Menge zuzugebender Spurenelemente in Abhängigkeit von der Differenz des Richtwertes und der ermittelten Konzentration ermittelt. Bei einer großen Differenz wird eine entsprechend große Menge der Spurenelemente und bei einer geringen Differenz eine entsprechend geringe Menge der Spurenelemente in Zeitabständen zugegeben. Zum Ausgleich von Verlusten der Spurenelemente wird gemäß einer weiteren Ausgestaltung die zuzugebende Menge der Spurenelemente unter Berücksichtigung der mit den Fermentationsrückständen im Biogasreaktor entnommenen Spurenelemente ermittelt.

Gemäß einer Ausgestaltung wird einmalig dem Biogasreaktor eine Menge Spurenelemente zugeführt, die so bemessen ist, daß eine sofortige Anhebung auf das endgültige Niveau der Spurenelemente erfolgt. In Zeitabständen kann die Gabe wiederholt werden. Sie kann insbesondere nach Ablauf eines Teils der Verweilzeit oder etwa nach Ablauf der Verweilzeit erneut in dem Biogasreaktor gegeben werden.

Gemäß einer weiteren Ausgestaltung wird anfänglich im Biogasreaktor eine Menge Spurenelemente zugegeben, die geringer ist, als der Bedarf. Die Zugabe wird später dem Bedarf angepaßt. Hierdurch kann sich das mikrobiologische System im Biogasreaktor allmählich an die neuen Bedingungen anpassen.

Zugrunde zu legen ist jeweils der Bedarf bezogen auf den Zeitraum, für den die Zugabe erfolgt. Der Zeitabschnitt, in dem eine den Bedarf unterschreitende Menge Spurenelemente zugegeben wird, ist vorzugsweise geringer als die Verweilzeit des Gärsubstrats im Biogasreaktor, die beispielsweise 1 bis 3 Monate beträgt. Gemäß einer Ausgestaltung wird anfänglich binnen einer bis zwei Wochen nur ein Teil der zuzugebenden Menge Spurenelemente zugegeben.

Gemäß einer weiteren Ausgestaltung werden die Spurenelemente in einer gut löslichen Form in den Biogasreaktor eingebracht. Gemäß einer weiteren Ausgestaltung werden sie gleichmäßig im Biogasreaktor verteilt. Hierdurch kann eine Überschuß- und Mangelsituation in den einzelnen Zonen des Biogasreaktors vermieden werden.

Gemäß einer Ausgestaltung werden die Spurenelemente kontinuierlich oder einmalig oder wiederholt zugegeben (z.B. in gleichen oder verschiedenen Zeitabständen und/oder in gleichen oder verschiedenen Mengen). Sie werden z.B. durch einmalige oder wiederholte Zugabe eines Depots, das Spurenelemente über einen längeren Zeitraum freisetzt, zugegeben. Eine einmalige Zugabe von Spurenelementen kann z.B. erfolgen, um die Biogaserzeugung im Biogasreaktor kurzfristig anzuheben. Langfristig kann dann die Biogaserzeugung durch eine geänderte Fütterung mit Biomasse auf hohem Niveau gehalten werden. Eine kontinuierliche oder wiederholte Zugabe von Spurenelementen kann z.B. erfolgen, wenn ein Spurenelementemangel der zugefütterten Biomasse langfristig ausgeglichen werden muß.

Die Zugabe der Spurenelemente kann in verschiedenen Zeitabständen erfolgen. Gemäß einer Ausgestaltung des Verfahrens erfolgt sie täglich oder in Abständen von mehreren Tagen. Gemäß einer anderen Ausgestaltung erfolgt sie in Zeitabständen, die etwa der Verweilzeit (z.B. 1 bis 3 Monate) der Biomasse im Biogasreaktor entsprechen. Diese Zeitabstände sind bevorzugt die maximalen Zeitabstände zwischen den Zugaben, weil angenommen werden kann, daß innerhalb der Verweilzeit die zugesetzten Spurenelemente im wesentlichen verbraucht bzw. dem Fermenter entnommen werden. Auch ist eine Zugabe in wechselnden Zeitabständen möglich.

Erfolgen die einzelnen Prozessschritte des Biogasprozesses in räumlich getrennten Behältern bzw. Biogasreaktoren, so kann durch die jeweilige Zugabe dem unterschiedlichen Bedarf der in den einzelnen Biogasreaktoren vorliegenden Bakteriengattungen Rechnung getragen werden.

Gemäß einer Ausgestaltung wird dem Biogasreaktor ein verschiedene Spurenelemente umfassender Zusatz zugegeben. Der Zusatz ist beispielsweise eine Mischung der verschiedenen Spurenelemente in flüssiger oder fester Form, wobei ein fester Zusatz in Form eines Pulvers oder in Form eines Granulates oder mindestens eines anderen Festkörpers zugesetzt werden kann, der schnell oder allmählich im Gärsubstrat zerfällt bzw. sich darin auflöst bzw. Spurenelemente freisetzt.

Gemäß einer Ausgestaltung wird der Zusatz speziell in Abhängigkeit von den Richtwerten und den ermittelten Konzentrationen hergestellt. Dem Biogasreaktor wird also ein speziell dem Bedarf angepasster Zusatz zugegeben, und zwar kontinuierlich, einmalig oder wiederholt.

Gemäß einer anderen Ausgestaltung werden mehrere Spurenelemente umfassende Zusätze mit verschiedenen Mengenverhältnissen der Spurenelemente hergestellt und wird von diesen Zusätzen derjenige dem Biogasreaktor zugeführt, dessen Zusammensetzung der mit Hilfe der Richtwerte und der ermittelten Konzentrationen ermittelten Zusammensetzung des dem Biogasreaktor zuzugebenden Zusatzes am nächsten kommt. Bei dieser Verfahrensvariante werden also verschiedene Standard-Zusätze bereitgehalten, unter denen derjenige im Bedarfsfalle ausgewählt wird, der am besten für den Ausgleich eines Mangels an Spurenelemente im Biogasreaktor geeignet ist. Dieser ausgewählte Zusatz wird kontinuierlich, einmalig oder wiederholt dem Biogasreaktor zugegeben.

Nachfolgend wird das Verfahren der Analyse der Spurenelemente mittels ICP-Analyse näher erläutert:

Probenahme:

Es wird aus dem zu untersuchenden Fermenter eine homogene Probe gezogen, so dass die Zusammensetzung in der Probe identisch ist mit der gesamten Zusammensetzung des Fermenterinhaltes. Die Probemenge sollte insgesamt rund 2kg betragen.

Bei jedem Aufarbeitungsschritt der Probe ist für eine ausreichende Durchmischung (Homogenität) zu sorgen.

Probenaufarbeitung:

Von der Probe werden rund 600 g in eine Aluschale, welche mit Backpapier ausgelegt ist eingewogen und diese dann für mindestens 48 Stunden bei 65 °C in einem Umlufttrockenschrank getrocknet. Die Probe aus dem Fermenter wird zunächst bei 65 °C getrocknet, um ein Material zu erhalten, welches sich lagern und verarbeiten läßt. Der Gewichtsverlust wird erfasst, indem das Probengefäß sowie die Probeneinwaage vor und nach der Trocknung gewogen werden.

*Berechnung der 65°C-Trockenmasse (kurz TM) in %:*

$$\%TM(65°C) = \text{Probenrückwaage nach Trocknung / Probeneinwaage vor Trocknung x } 100\%$$

Das gesamte trockene Probenmaterial wird in einer Mühle (Feinheit 1 mm Siebdurchgang) zermahlen.

Das bei 65 °C getrocknete Material enthält noch gewisse Restmengen an Wasser. Von dem bei 65 °C getrockneten und gemahlenen Material wird eine Bestimmung der Trockenmasse bei 105 °C durchgeführt, indem der Gewichtsverlust bei 4 Stunden Trocknung bei 105 °C ermittelt wird.

*Berechnung der 105°C-TM in %:*

$$\%TM(105°C) = \text{Probenrückwaage nach Trocknung / Probeneinwaage vor Trocknung x } 100\%$$

Der Restwassergehalt ist die Differenz von %TM(105°C) zu 100%.

*Berechnung der gesamten Trockenmasse im Fermenter:*

$$\%TM_{Fermenter} = \%TM(105°C) \times \%TM(65°C) / 100\%$$

Probenaufschluss:

Von dem homogenen Probenmaterial werden exakt 3g in ein Quarzröhrchen eingewogen und auf einer Heizplatte so stark erhitzt, dass das organische Material beginnt zu verkohlen. Sobald die Probe nicht mehr raucht, kommt das Quarzröhrchen in einen Muffelofen um dort für mindestens 32Stunden bei 550°C zu veraschen.

In das ausgekühlte Quarzröhrchen gibt man 5 ml 65%-ige Salpetersäure, sowie 0,5 ml 30 %-ige Wasserstoffperoxid-Lösung und stellt das Quarzröhrchen in einen Mikrowellendruckbehälter, um die Probe anschließend in der Mikrowelle aufzuschließen. Die Bedingungen des Mikrowellenauf-schlusses sind so zu wählen, dass eine maximale Menge an Spurenelementen in Lösung gehen (ca. 7,5 min bei 600 Watt).

Die aufgeschlossene Probe wird mit entionisiertem Wasser in einen Messkolben, in der Regel ein 50ml-Messkolben, überführt und bis zur Messmarke aufgefüllt.

Messung der Elemente mittels ICP-Spektrometer:

Eventuell vorhandene ungelöste Bestandteile werden abfiltriert und die Lösung anschließend mittels ICP-OES Spektrometer vermessen. ICP-OES heißt induktiv gekoppeltes Plasma mit Auswertung des optischen Emissionsspektrums. Dies ist ein gängiges Messverfahren zur Bestimmung von gelösten Elementen, wobei die Probelösung in einer ca. 5000-8000°Kelvin heißen Flamme (erzeugt durch induktiv gekoppeltes Plasma) gepumpt wird. Die in der Probenlösung enthaltenen Elemente emittieren dann die für jedes Element typischen Spektrallinien, die optisch aufgearbeitet und ausgelesen werden können. Das Gerät verfügt über eine Kalibrierung die mittels verschiedener Standardlösungen, mit den Elementen, die der Matrix der Fermenterinhalte sehr ähnlich sind, erstellt wurde. Mit Hilfe der Kalibrierung wird für jedes Element quantitativ der Gehalt errechnet.

Es werden quantitativ folgende Elemente untersucht:

Natrium, Calcium, Kalium, Magnesium, Schwefel, Phosphor, Kupfer, Bor, Mangan, Zink, Nickel, Kobalt, Molybdän, Selen, Eisen, Wolfram.

In Zukunft ist es denkbar auch die Gehalte weiterer Elemente zu erfassen, sofern ein Zusammenhang zwischen Konzentration des Elementes und der Funktion des Fermenters erwartet wird.

Berechnung der Elementanteile in TM:

Anhand der ICP-Analyse erhält man für die untersuchten Elemente deren Gehalte in mg/l und rechnet unter Berücksichtigung der Einwaage, der Verdünnungen und des Gehaltes an Restfeuchte auf die Gehalte in der Trockenmasse um. Man erhält somit für jedes untersuchte Spurenelement (allgemein ME) den Gehalt im Fermenterschlamm bezogen auf die Trockenmasse:

$$\text{Konz. (Me)}_{Fermenter} \text{ in mg/kgTM}$$

Darlegung der Berechnung der Zugabe-Mengen an Spurenelementen für einen optimalen Betrieb der Biogasanlagen

Allgemein:

Mit Hilfe der ermittelten Gehalte der verschiedenen Spurenelemente und der Erkenntnis, welche Gehalte für einen optimalen Biogasprozess notwendig sind, lässt sich für jedes einzelne Element errechnen, ob der Gehalt des jeweiligen Spurenelementes ausreichend vorhanden ist, oder ob es ein Defizit gibt. Gibt es ein Defizit muss dieses Defizit ausgeglichen werden, indem gut lösliche und hochverfügbare Spurenelemente als Salz zugegeben werden. Im Fermenter muss eine gute homogene Verteilung der Spurenelement-Zusätze gewährleistet sein.

Me steht allgemein für alle Spurenelemente. Die folgende Berechnung muss für alle notwendigen Spurenelemente einzeln durchgeführt werden.

Berechnung des Defizits:

$$\text{Konz. (Me)}_{Optimum} - \text{Konz. (Me)}_{Fermenter} = \text{Defizit}_{Me} \text{ (mg/kgTM)}$$

$$\text{Konz. (Me)}_{Optimum} \text{ in mg/kg TM} = \text{optimale Konzentration des Spurenelementes Me}$$

$$\text{Konz. (Me)}_{Fermenter} \text{ in mg/kg TM} = \text{ermittelte Konzentration des Spurenelementes Me}$$

Ist das Defizit negativ, also Konz. $(Me)_{Optimum}$ < Konz. $(Me)_{Fermenter}$ ist keine Zugabe notwendig.

Ist das Defizit positiv, also Konz. $(Me)_{Optimum}$ > Konz. $(Me)_{Fermenter}$ ist Zugabe notwendig

Berechnung Defizit-Ausgleich:

Wurde für ein Spurenelement ein positives Defizit ermittelt, muss dieses Defizit durch Zugabe ausgeglichen werden. Der Ausgleich wird zur Hälfte des tatsächlichen Defizits berechnet und über 7 Tage verteilt zugegeben, damit sich das mikrobiologische System langsam an die neuen Bedingungen anpassen kann. Für die Ermittlung kann praktischerweise davon ausgegangen werden, dass der Fermenterinhalt in ($m^3$) gleich der Masse in (to) ist.

*Zugabe Spurenelement in 7 Tagen für 50%-Ausgleich des Defizits:*

$$\text{Fermenterinhalt (to)} \times \%TM_{Fermenter} \, (\%) \times Defizit_{Me} \, (mg/kgTM) \times 0{,}5 \, / \, 100\% =$$

$$Zugabe_{Me\,50\%Soll} \, (g)$$

Da das Spurenelement in Form eines Salzes oder einer Salzvormischung eingesetzt wird, muss die Zugabe des Spurenelementes auf die Zugabe des Spurenelementsalzes umgerechnet werden, indem der Gehalt des Spurenelementes im Salz oder der Salzvormischung (% Me-Anteil Salz) berücksichtigt wird.

*Zugabe Spurenelementsalz in 7 Tagen für 50%-Ausgleich des Defizits:*

$$Zugabe_{Me\,50\%Soll} \, (g) \, / \, \% \text{ Me-Anteil Salz} \times 100\% = Zugabe_{Me\text{-}Salz\,50\%Soll} \, (g)$$

Berechnung Austragverluste:

Nach den 7 Tagen wird die Menge an Spurenelementen zugegeben, die täglich durch den Austrag aus dem Fermenter an Spuren verloren gehen, und nicht durch Substratfütterung ausgeglichen werden. Dieser tägliche Austrag führt bei unveränderter Substratfütterung über einen Zeitraum von mehreren Tagen genau zu dem anfangs erwähnten Defizit an Spurenelementen.

Die Berechnung erfolgt über die hydraulische Verweilzeit (HRT) im Fermenter, die angibt, wie lange eine zugegebene Substanz im Mittel im Fermenter verbleibt. Da in den ersten 7 Tagen nur 50% des Defizits ausgeglichen wurden, jetzt jedoch davon ausgegangen wird, dass das ganze Defizit anteilig ausgetragen wird, wird erreicht, dass sich die Konzentration des Spurenelementes langsam an den optimalen Bedarf annähert.

*Tägliche Zugabe Spurenelement für Ausgleich der Austragverluste:*

$$\text{Konz. } (Me)_{Optimum} - \text{Konz. } (Me)_{Fermenter} = Defizit_{Me} \, (mg/kgTM)$$

$$16{,}0 - 4{,}3 = 11{,}7 \, mg/kgTM = Defizit_{Ni}$$

Das Defizit positiv, also Konz. $(Ni)_{Optimum}$ > Konz. $(Ni)_{Fermenter}$ ist Zugabe notwendig

Berechnung Defizit-Ausgleich:

*Zugabe Spurenelement in 7 Tagen für 50%-Ausgleich des Defizits:*

$$\text{Fermenterinhalt (to)} \times \%TM_{Fermenter} \, (\%) \times Defizit_{Me} \, (mg/kgTM) \times 0{,}5 \, / \, 100\% =$$

$$Zugabe_{Me\,50\%Soll} \, (g)$$

$$2.500to \times 8{,}7\% \times 11{,}7 \, mg/kgTM \times 0{,}5 \, / \, 100\% = 1272{,}5 \, g \, Nickel = Zugabe_{Me\,50\%Soll} \, (g)$$

*Zugabe Spurenelementsalz in 7 Tagen für 50%-Ausgleich des Defizits:*

$$Zugabe_{Me\ 50\%Soll}\ (g)\ /\ \%\ Me\text{-}Anteil\ Salz\ x\ 100\% = Zugabe_{Me\text{-}Salz\ 50\%Soll}\ (g)$$

$$1272{,}5\ g\ Ni\ /\ 22{,}35\%\ Ni\ im\ Salz\ x\ 100\% = 5693{,}4\ g\ Nickelsulfat\ Hexahydrat$$

$$= Zugabe_{Me\text{-}Salz\ 50\%Soll}$$

Berechnung Austragverluste:

*Tägliche Zugabe Spurenelement für Ausgleich der Austragverluste:*

$$Fermenterinhalt\ (to)\ x\ TM_{Fermenter}\ (\%)\ x\ Defizit_{Me}\ (mg/kgTM)\ /\ 100\%\ /\ HRT$$

$$(d) = Zugabe_{Me\ täglich}\ (g)$$

Da das Spurenelement in Form eines Salzes oder einer Salzvormischung eingesetzt wird, muss die Zugabe des Spurenelementes auf die Zugabe des Spurenelementsalzes umgerechnet werden, indem der Gehalt des Spurenelementes im Salz oder der Salzvormischung (% Me-Anteil Salz) berücksichtigt wird.

*Tägliche Zugabe Spurenelementsalz für Ausgleich der Austragverluste:*

$$Zugabe_{Me\ täglich}\ (g)\ /\ \%\ Me\text{-}Anteil\ Salz\ x\ 100\% = Zugabe_{Me\text{-}Salz\ täglich}\ (g)$$

**Beispielberechnung:**

Um das konkrete Vorgehen zu verdeutlichen wird ein Beispiel berechnet anhand des Spurenelementes Nickel.

*Annahmen für das Beispiel:*

*Konz. (Ni)$_{Fermenter}$ = 4,3mg/kgTM laut Analyse des Fermenters*
*Fermenterinhalt = 2.500m3 bzw. 2.500to*
*Mittlere Verweilzeit (HRT) = 63Tage*
*TM Fermenter = 8, 7%*
*Zugabe als Nickelsulfat Hexahydrat mit 22,35% Nickelgehalt*

Berechnung des Defizits:

*Für Nickel sind 4 - 30 mg/kg TM als optimal erarbeitet worden*

$$Konz.\ (Ni)_{Optimum}\ =\ 16{,}0\ mg/kg\ TM\ =\ optimale\ Konzentration\ des$$

$$Spurenelementes\ Ni$$

$$Fermenterinhalt\ (to)\ x\ TM_{Fermenter}\ (\%)\ x\ Defizit_{Me}\ (mg/kgTM)\ /\ 100\%\ /\ HRT$$

$$(d) = Zugabe_{Me\ täglich}\ (g)$$

$$2.500\text{to} \times 8{,}7\% \times 11{,}7 \text{ mg/kgTM} \, / \, 100\% \, / \, 63\text{d} = 40{,}4 \text{ g Ni} = \text{Zugabe}_{\text{Me täglich}}$$

*Tägliche Zugabe Spurenelementsalz für Ausgleich der Austragverluste:*

$$\text{Zugabe}_{\text{Me täglich}} (g) \, / \, \% \text{ Me-Anteil Salz} \times 100\% = \text{Zugabe}_{\text{Me-Salz täglich}} (g)$$

$$40{,}4 \text{ g} \, / \, 22{,}35\% \times 100\% = 108{,}8 \text{ g Nickelsulfathexahydrat} = \text{Zugabe}_{\text{Me-Salz täglich}}$$

Berechnung Spurenelementmischung:

Da jedes Spurenelement welches sich im Defizit befindet zugegeben werden soll, wird aus den verschiedenen Spurenelementsalzen eine Spurenelementmischung berechnet, die die notwendigen Spurenelemente in dem Verhältnis enthält, wie sie aus den Zugabemengen berechnet wurden. Es wird anhand der Betriebsdaten des Biogasbetreibers eine Zugabeempfehlung berechnet, so dass die errechneten Zugabemengen erreicht werden. Gegebenenfalls wird ein Füllstoff zugemischt, um eine bessere Handhabbarkeit der Spurenelementmischung zu erzielen.
*Zugabe Spurenelementmischung in 7 Tagen für 50%-Ausgleich des Defizits:*

$$\text{Summe aller Zugabe}_{\text{Me-Salz 50\%Soll}} (g) + \text{Füllstoff} (g) = \text{Zugabe }_{\Sigma \text{ Me Mischung 50\%-Defizit}} \text{ über 7 Tage}$$

Für eine gleichmäßige Verteilung auf 7 Tage muss die Menge durch 7 Tage geteilt werden:

*Tägliche Zugabe Spurenelementmischung über 7 Tage für 50%-Ausgleich des Defizits:*

$$\text{Zugabe }_{\Sigma \text{ Me Mischung 50\%-Defizit}} \text{ über 7 Tage} \, / \, 7 \text{ Tage} = \text{Zugabe }_{\Sigma \text{ Me Mischung 50\%-Defizit}} \text{ täglich}$$

Analog wird mit der Zugabe für den Ausgleich der Austragverluste verfahren:

*Tägliche Zugabe Spurenelementmischung für Ausgleich der Austragverluste:*

$$\text{Summe aller Zugabe}_{\text{Me-Salz täglich}} (g) + \text{Füllstoff} (g) = \text{Zugabe }_{\Sigma \text{ Me Mischung täglich}}$$

Ergebnisse von Praxisuntersuchungen:

Beispiel 1:

Eine güllefrei betriebene Biogasanlage, die bereits seit vier Monaten eine Prozesshemmung mit stark erhöhten Säurewerten und Fos/Tac-Werten (welcher das Verhältnis flüchtiger organischer Säuren und dem anorganischen Kohlenstoff als Maß der Pufferkapazität beschreibt) sowie eine in der Folge reduzierte Gasproduktion aufzeigte wurde mit einer speziell an diese Biogasanlage angepassten Spurenelementgabe beschickt. Die Fütterung bestand aus Maissilage, Getreidekorn und Grassilage. Nach Zugabe der Spurenelemente erfolgte innerhalb von 24-72h sowohl ein Anstieg der Gasqualität, als auch der gebildete Gasmenge aufgrund eines Abbaus der zuvor aufgrund der Prozesshemmung angereicherten Säuren. Trotz einer in der Folge erhöhten Fütterung zeigten die Analysewerte des Gärsubstrates eine stetige Verbesserung Prozessbedingungen. Die Säuren reduzierten sich in der Folge von vormals kritischen, eine Prozesshemmung anzeigenden Konzentrationen auf extrem niedrige Gehalte, welche einen stabilen Prozess belegen.

Insgesamt stieg die Leistung der Biogasanlage innerhalb der ersten 10 Tage von 600 kW auf 840 kW, was einer Leistungssteigerung von 40% entspricht.

Die Entwicklung der Fos/Tac-Werte und des Energieertrages vor und nach Einsatz einer Spurenelementezugabe sind im anliegenden Diagramm dargestellt. Darin ist der Verlauf der Fos/Tac-Werte über der Zeit im Hauptfermenter (x), im Nachgärer 1 (Quadrate) und im Nachgärer 2 (Rauten) dargestellt. Ferner ist die Gesamtleistung der Motoren (Dreiecke) dargestellt. Die jeweiligen Meßwerte sind durch Kurven verbunden. Es ist gut erkennbar, daß die Leistung der Biogasanlage innerhalb von 10 Tagen nach der Spurenelementezugabe um 40 % ansteigt.

Zu den Fos/Tac-Werten ist folgendes anzumerken:

Der Fos/Tac hat sich bei der Analyse von Biogasfermentern bewährt und wird bei praktisch allen Untersuchungen mit durchgeführt.

Durch Titration mit einer bestimmten Säure kann die Summe der organischen Säuren (Fos) und die Summe des Carbonatpuffers (Tac) ermittelt werden.

Das Verhältnis Fos/Tac daraus sollte unter 0,3 liegen, was bedeutet, dass das Verhältnis zwischen Säure und Puffer ausgewogen ist.

Steigt der Wert über 0,4 sind zu viele Säure für den vorhandenen Cabonatpuffer vorhanden. Dies ist ein eindeutiges bekanntes Anzeichen für einen nicht optimalen Biogasprozess, häufig dadurch ausgelöst, daß die Säuren nicht schnell genug oder nicht ausreichend genug abgebaut werden.

Es werden 20ml zentrifugierter Fermenterprobe mit ca. 80ml Wasser verdünnt und während des Rührens wird mit 0,1 n Schwefelsäure titriert und dabei der pH-Wert gemessen.

Man notiert den Verbrauch an Schwefelsäure (ml 0,1n Schwefelsäure) bis zum pH-Wert von 5,0 (=α) und titriert weiter bis zum pH-Wert von 4,4. Man notiert den Verbrauch an Schwefelsäure (ml 0,1n Schwefelsäure) von pH 5,0 bis pH 4,4 (=β).

$$\text{Tac} = \alpha \times 250$$

$$\text{Fos} = (\beta \times 1{,}66 - 0{,}15) \times 500$$

$$\text{Fos/Tac} = \text{Fos} : \text{Tac}$$

Beispiel 2:

In einer in Kofermentation von Rindergülle mit Maissilage, Sudangras und Weizenkorn betriebenen Biogasanlage waren nur Faulraumbelastungen von 2 kg organischer Substanz je Kubikmeter Fermentervolumen realisierbar. Bei einer Anhebung der Fütterung reicherten sich die kurzkettigen Fettsäuren, welche üblicherweise in weiteren Schritten zu Methan und Kohlendioxid abgebaut werden, an und es trat eine Hemmung des Abbaus mit drohendem Zusammenbruch der Biogasbildung ein. Die Biogasanlage verfügt über zwei identische Fermenter, die gleich beschickt wurden. Einer dieser Fermenter wurde mit Spurenelementen behandelt, der zweite als Kontrolle wie gehabt betrieben. Nach der Spurenelementbehandlung zeigte sich ein schneller Anstieg der Biogasmenge und Qualität, während der unbehandelte Fermenter keine Veränderungen aufzeigte. Die erhöhte Gasmenge resultierte aus einem Abbau der organischen Säuren, welche aufgrund der nun nicht mehr gehemmten Biologie zu den Endprodukten Methan und Kohlendioxid abgebaut werden konnten (Tabelle 1). Eine anschließende Erhöhung der Zufuhr an organischer Substanz resultierte in einer erhöhten Gasproduktion, jedoch ohne weitere Anzeichen einer Hemmung. Der zunächst als Kontrolle weiterhin ohne Spurenelementzugabe betriebene Fermenter zeigte trotz erheblich geringerer Belastung nur eine geringfügige Verbesserung der Analysewerte.

**Tabelle 1:** Entwicklung der flüchtigen Fettsäuren einer Biogasanlage nach einer Spurenelementzugabe im Vergleich zur Kontrollvariante; (Zielwerte eines stabilen Biogasprozessen: Verhältnis Essigsäure zu Propionsäure > 2:1; Propionsäure < 1000 mg / kg FM)

| Datum | Fermenter 1 | | | Fermenter 2 | | |
|---|---|---|---|---|---|---|
| | Acetat | Propionat | Butyrat | Acetat | Propionat | Butyrat |
| | [mg / kg FM] | [mg / kg FM] | [mg / kg FM] | [mg / kg FM] | [mg / kg FM] | [mg / kg FM] |

(fortgesetzt)

| Datum | Fermenter 1 | | | Fermenter 2 | | |
|---|---|---|---|---|---|---|
| | Acetat | Propionat | Butyrat | Acetat | Propionat | Butyrat |
| 20.02.2007 | 1.385 | 4.470 | 701 | 1.055 | 4.484 | 586 |
| 10.02.2007 | Zugabe der Spurenelemente | | | Keine Zugabe von Spurenelementen | | |
| 05.03.2007 | 679 | 1216 | 370 | 1.1016 | 3.805 | 529 |
| 12.03.2007 | 203 | 76 | 2 | 738 | 3.109 | 455 |

Spurenelementversorgung der Anlage 2

| Element | Ausgangskonzentration | Zugabemenge |
|---|---|---|
| | [mg / kg TS] | [mg / kg TS] |
| Nickel | 2,3 | 14,2 |
| Kobalt | 0,5 | 0,3 |
| Molybdän | 1,5 | 1,3 |
| Eisen | 826 | 769 |
| Mangan | 131 | Keine Zugabe |
| Kupfer | 19,3 | Keine Zugabe |
| Selen | 0,22 | Keine Zugabe |
| Wolfram | Nicht erfasst | Keine Zugabe |
| Zink | 138 | Keine Zugabe |

Die erfindungsgemäß bereitgestellten Richtwerte der Konzentrationen der Spurenelemente Nickel, Kobalt, Molybdän, Eisen sowie deren Optimalbereich und die Grenzwerte für die Aufbringung auf landwirtschaftliche Flächen sind in der nachfolgenden Übersicht zusammengestellt, ebenso wie, gemäß einer Ausgestaltung, die Richtwerte für Mangan, Kupfer, Selen, Wolfram und Zink:

**Richtwerte der optimalen Spurenelementkonzentrationen**

| Element | Optimalbereich | Sollbereich | Grenzwerte |
|---|---|---|---|
| | [mg / kg TS] | [mg / kg TS] | [mg / kg TS] |
| Nickel | 16 | 4 - 30 | 50 (30)*) |
| Kobalt | 1,8 | 0,4 - 5 | |
| Molybdän | 4 | 1 - 10 | |
| Eisen | 2400 | 1500 - 3500 | |
| Mangan | 300 | 100 - 1500 | |
| Kupfer | 40 | 10 - 80 | 100 *) |
| Selen | 0,5 | 0,05 - 4 | |
| Wolfram | 0,6 | 0,1 - 30 | |

# EP 1 997 901 B1

(fortgesetzt)

| Element | Optimalbereich | Sollbereich | Grenzwerte |
|---------|----------------|-------------|------------|
| Zink | 200 | 30 - 400 | 400 *) |
| *1) Grenzwerte der BioAbfV zur Aufbringung auf landwirtschaftliche Flächen, In Klammern: Verordnung über Umweltgefährdende Stoffe (Stoffverordnung StoV), Änderung vom 26. März 2003 im Namen des schweizerischen Bundesrates | | | |

Vorhandene Grenzwerte werden von den Richtwerten jeweils deutlich unterschritten.

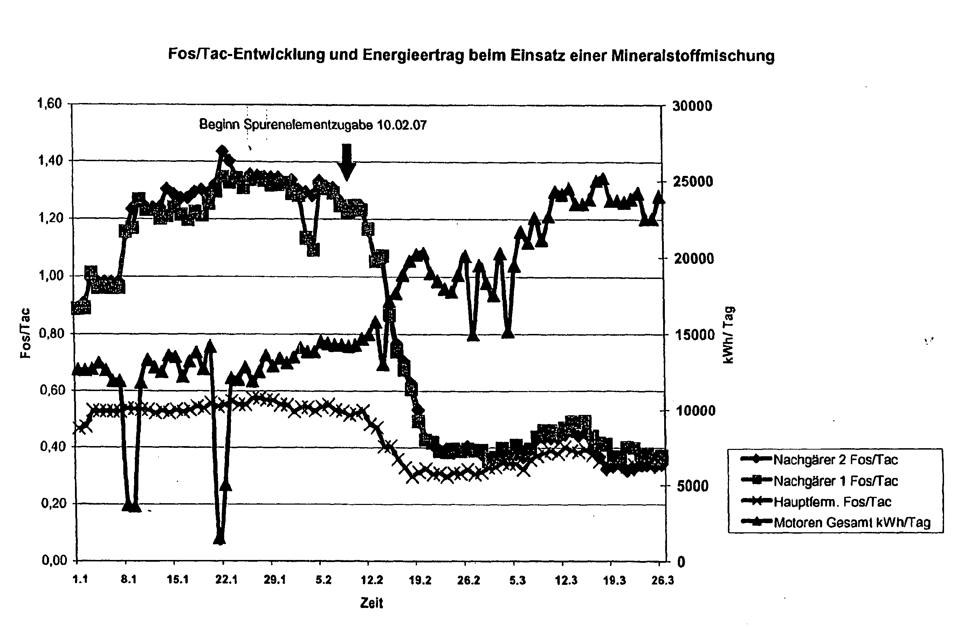

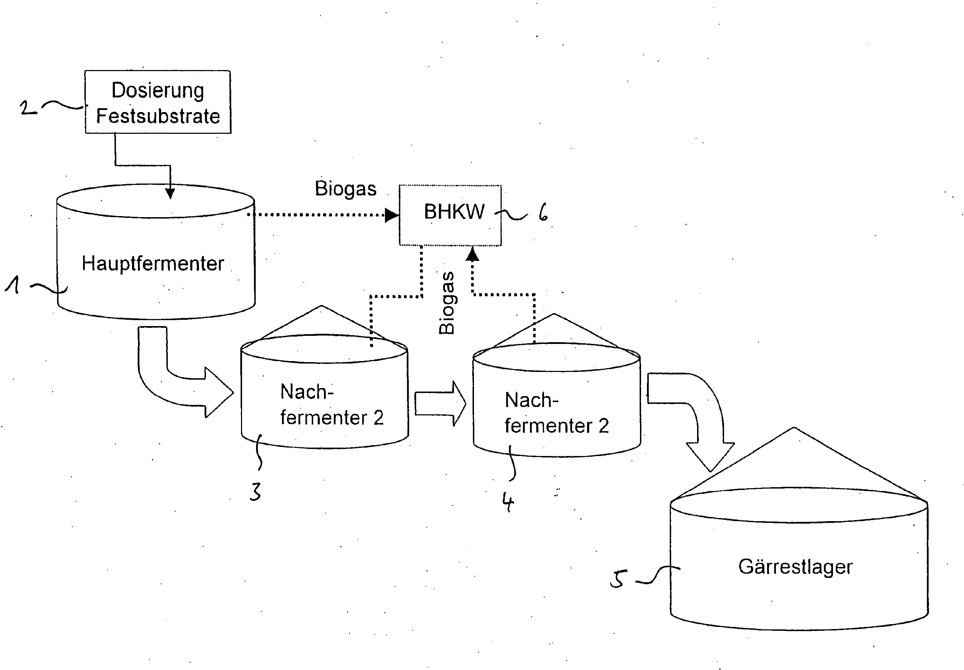

In der Zeichnung ist grobschematisch eine Biogasanlage gezeigt, der erfindungsgemäß Spurenelemente zum Ausgleich eines Mangels an Spurenelementen zugeführt werden können.

Die Biogasanlage umfaßt eine Hauptfermenter 1, in den Festsubstrate über eine Dosierung 2 eindosierbar sind. Dem Hauptfermenter 1 nachgeschaltet ist ein Nachfermenter 3 und diesem ist wiederum ein weiterer Nachfermenter 4 nachgeschaltet. Vom weiteren Nachfermenter 4 gelangen Gärreste in ein Gärrestlager 5.

Aus dem Hauptfermenter 1, dem Nachfermenter 3 und dem weiteren Nachfermenter 4 werden die Biogase einem Blockheizkraftwerk 6 zugeführt, das elektrischen Strom und Heizungswärme erzeugt.

In dem Hauptfermenter 1 erfolgt ein Teil der Biogaserzeugung von der Hydrolyse bis hin zur Methanbildung. Dort wird auch das meiste Biogas abgezogen. Eine Restmethanbildung unter weiterem Abbau der Biomasse erfolgt in den Nachfermentern 3 und 4. Ein Spurenelementemangel wird ausgeglichen, indem über die Dosierung 2 der Feinsubstrate Spurenelemente der Biogasanlage zugeführt werden.

## Patentansprüche

1. Verfahren zur Biogaserzeugung aus Biomasse in einem Biogasreaktor, bei dem

   - mindestens ein Richtwert für die Konzentration mindestens eines der Spurenelemente Nickel, Kobalt, Molybdän, Eisen in einem Biogasreaktor für eine effektive Biogaserzeugung bereitgestellt wird,
   - in dem Biogasreaktor aus Biomasse Biogas erzeugt wird,
   - die Konzentration mindestens eines Spurenelementes in der Biomasse im Biogasreaktor ermitttelt wird;
   - im Falle einer Unterschreitung des Richtwertes durch die ermittelte Spurenelementekonzentration fehlende Spurenelemente dem Biogasrcaktor zugegeben werden,
   - die Richtwerte für Nickel 4 bis 30 mg/kg TS und/oder für Kobalt 0,4 bis 5 mg/kg TS und/oder für Molybdän 1,0 bis 10,0 mg/kg TS und/oder für Eisen 1500 bis 3500 mg/kg TS betragen.

2. Verfahren nach Anspruch 1, bei dem Richtwerte für die Konzentration der Spurenelemente Nickel und/oder Kobalt und/oder Molybdän und/oder Eisen bereitgestellt und die Konzentration der Spurenelemente Nickel und/oder Kobalt und/oder Molybdän und/oder Eisen im Biogasreaktor ermittelt werden.

3. Verfahren nach einem der Ansprüche 1 oder 2, bei dem die Richtwerte für Nickel mindestens 10 und/oder maximal 25 mg/kg TS und/oder für Kobalt mindestens 1,0 mg/kg TS betragen.

4. Verfahren nach einem der Ansprüche 1 bis 3, bei dem Richtwerte für die Konzentrationen der Spurenelemente Mangan und/oder Kupfer und/oder Selen und/oder Wolfram und/oder Zink bereitgestellt und die Konzentrationen der Spurenelemente Mangan und/oder Kupfer und/oder Selen und/oder Wolfram und/oder Zink im Biogasreaktor ermittelt werden.

5. Verfahren nach Anspruch 4, bei dem die Richtwerte für Mangan 100 bis 1500 mg/kg TS und/oder für Kupfer 10 bis 80 mg/kg TS und/oder für Selen 0,05 bis 4 mg/kg TS und/oder für Wolfram 0,1 bis 30 mg/kg TS und/oder für Zink 30 bis 400 mg/kg TS betragen.

6. Verfahren nach Anspruch 5, bei dem die Richtwerte für Mangan mindestens 250 und/oder maximal 350 mg/kg TS und/oder für Kupfer mindestens 30 und/oder maximal 50 mg/kg TS und/oder für Selen mindestens 0,3 und/oder maximal 0,7 mg/kg TS und/oder für Wolfram mindestens 0,4 und/oder maximal 0,8 mg/kg TS und/oder für Zink mindestens 150 und/oder maximal 250 mg/kg TS betragen.

7. Verfahren nach einem der Ansprüche 1 bis 6, bei dem die biologische Verfügbarkeit der im Biomaterial im Biogasreaktor enthaltenen Spurenelemente erhöht wird.

8. Verfahren nach Anspruch 7, bei dem dem Biogasreaktor ein die biologische Verfügbarkeit der Spurenelemente erhöhender Zusatz zugeführt wird.

9. Verfahren nach Anspruch 8, bei dem der Zusatz Eisen enthält.

10. Verfahren nach einem der Ansprüche 7 bis 9, bei dem nach dem Erhöhen der biologischen Verfügbarkeit der Spurenelemente mindestens ein Spurenelement zugegeben wird.

11. Verfahren nach einem der Ansprüche 7 bis 10, bei dem nach dem Erholen der biologischen Verfügbarkeit der Spurenelemente die Konzentration mindestens eines Spurenelementes im Biomaterial ermittelt und ein Mangel des Spurenelementes durch Zugabe desselben kompensiert wird.

12. Verfahren nach einem der Ansprüche 1 bis 11, bei dem die Konzentration mindestens eines Spurenelementes in mindestens einer Probe aus dem Biogasreaktor durch ICP-Analyse ermittelt wird.

13. Verfahren nach einem der Ansprüche 1 bis 12, bei dem die Konzentration mindestens eines Spurenelementes im Biogasreaktor in Zeitabständen wiederholt ermittelt wird.

14. Verfahren nach einem der Ansprüche 1 bis 13, bei dem die zuzugebende Menge der Spurenelemente in Abhängigkeit von der Differenz des Richtwertes und der ermittelten Konzentration ermittelt wird.

15. Verfahren nach Anspruch 14, bei dem die zuzugebende Menge der Spurenelemente unter Berücksichtigung der mit den Fermentationsrückständen dem Biogasreaktor entnommenen Spurenelemente ermittelt wird.

16. Verfahren nach einem der Ansprüche 1 bis 15, bei dem anfänglich nur ein Teil der zuzugebenden Menge Spurenelemente zugegeben und später Mengen entsprechend dem Bedarf an zuzugebenden Spurenelemente zugegeben werden.

17. Verfahren nach Anspruch 16, bei dem anfänglich binnen einer bis zwei Wochen ein Teil der zuzugebenden Menge Spurenelemente zugegeben wird.

18. Verfahren nach einem der Ansprüche 1 bis 17, bei dem die Spurenelemente kontinuierlich oder einmalig oder wiederholt zugegeben werden und/oder durch einmalige oder wiederholte Zugabe eines Depots, das Spurenelemente über einen längeren zeitraum freisetzt, zugegeben werden.

19. Verfahren nach einem der Ansprüche 1 bis 18, bei dem dem Biogasreaktor ein verschiedene Spurenelemente umfassender Zusatz zugegeben wird.

20. Verfahren nach Anspruch 19, bei dem der Zusatz speziell in Abhängigkeit von den Richtwerten und den ermittelten Konzentrationen hergestellt wird.

21. Verfahren nach Anspruch 19, bei dem mehrere verschiedene Spurenelemente umfassende Zusätze mit verschiedenen Mengenverhältnissen der Spurenelemente hergestellt werden und von diesen Zusätzen derjenige dem Biogasreaktor zugeführt wird, dessen Zusammensetzung der mit Hilfe der Richtwerte und der ermittelten Konzentrationen ermittelten Zusammensetzung des dem Biogasreaktor zuzugebenden Zusatzes am nächsten kommt.

**Claims**

1. A method for producing biogas from biomass in a biogas reactor, wherein

  • at least one standard value is provided for the concentration of at least one of the trace elements nickel, cobalt, molybdenum, iron in a biogas reactor for efficient biogas production,

• biogas is produced from biomass in the biogas reactor,
• the concentration of at least one trace element in the biomass is determined in the biogas reactor, and
• in the event that the determined trace element concentration falls below the standard value, deficient trace elements are added to the biogas reactor,
• the standard values for nickel are 4 to 30 mg/kg DM and/or for cobalt 0,4 to 5 mg/kg DM and/or for molybdenum 1,0 to 10,0 mg/kg DM and/or for iron 1500 to 3500 mg/kg DM.

2. A method according to claim 1, wherein standard values are provided for the concentration of the trace elements nickel and/or cobalt and/or molybdenum and/or iron, and the concentration of the trace elements nickel and/or cobalt and/or molybdenum and/or iron is determined in the biogas reactor.

3. A method according to claim 1 or 2, wherein the standard values for nickel are at least 10 and/or at most 25 mg/kg DM and/or for cobalt at least 1,0 mg/kg DM.

4. A method according to any one of claims 1 to 3, wherein standard values are provided for the concentrations of the trace elements manganese and/or copper and/or selenium and/or tungsten and/or zinc, and the concentrations of the trace elements manganese and/or copper and/or selenium and/or tungsten and/or zinc in the biogas reactor are determined.

5. A method according to claim 4, wherein the standard values for manganese are 100 to 1500 mg/kg DM and/or for copper 10 to 80 mg/kg DM and/or for selenium 0,05 to 4 mg/kg DM and/or for tungsten 0,1 to 30 mg/kg DM and/or for zinc 30 to 400 mg/kg DM.

6. A method according to claim 5, wherein the standard values for manganese are at least 250 and/or at most 350 mg/kg DM and/or for copper at least 30 and/or at most 50 mg/kg DM and/or for selenium at least 0,3 and/or at most 0,7 mg/kg DM and/or for tungsten at least 0,4 and/or at most 0,8 mg/kg DM and/or for zinc at least 150 and/or at most 250 mg/kg DM.

7. A method according to any one of claims 1 to 6, wherein the biological availability of the trace elements that are contained in the biomaterial in the biogas reactor is increased.

8. A method according to claim 9, wherein an additive increasing the biological availability of the trace elements is supplied to the biogas reactor.

9. A method according to claim 8, wherein the additive contains iron.

10. A method according to any one of claims 7 to 9, wherein at least one trace element is added after the increasing of the biological availability of the trace elements.

11. A method according to any one of claims 7 to 10, wherein the concentration of at least one trace element in the biological material is determined after the increasing of the biological availability of the trace elements, and a shortage of the trace element is compensated by adding the same.

12. A method according to any one of claims 1 to 11, wherein the concentration of at least one trace element in at least one sample from the biogas reactor is determined by ICP analysis.

13. A method according to any one of claims 1 to 12, wherein the concentration of at least one trace element in the biogas reactor is repeatedly determined in time intervals.

14. A method according to any one of claims 1 to 13, wherein the amount of trace elements to be added is determined depending on the difference between the standard value and the determined concentration.

15. A method according to claim 14, wherein the amount of trace elements to be added is determined taking into account the trace elements that were taken out of the biogas reactor with the fermentation residues.

16. A method according to any one of claims 1 to 15, wherein only a part of the amount of trace elements to be added is added initially, and amounts corresponding to the need of trace elements to be added are added later.

**17.** A method according to claim 16, wherein a part of the amount of trace elements to be added is added initially within one to two weeks.

**18.** A method according to any one of claims 1 to 17, wherein the trace elements are added continuously or one-time or repeatedly, and/or are added by one-time or repeated addition of a depot which releases trace elements over a longer period of time.

**19.** A method according to any one of claims 1 to 18, wherein an additive comprising different trace elements is added to the biogas reactor.

**20.** A method according to claim 19, wherein the additive is specially produced depending on the standard values and the determined concentrations.

**21.** A method according to claim 19, wherein additives comprising plural different trace elements in different amount ratios of the trace elements are produced, and that one of these additives is supplied to the biogas reactor whose composition most approaches the composition of the additive to be added to the biogas reactor that was determined with the aid of the standard values and the determined concentrations.

**Revendications**

**1.** Procédé de production de biogaz à partir de la biomasse dans un réacteur de biogaz, dans lequel

- au moins une valeur indicative pour la concentration d'au moins un des éléments traces nickel, cobalt, molybdène, fer dans un réacteur de biogaz est fournie pour une production de biogaz efficace,
- du biogaz est produit dans le réacteur de biogaz à partir de la biomasse,
- la concentration d'au moins un élément trace dans la biomasse dans le réacteur de biogaz est déterminée ;
- des éléments traces manquants sont ajoutés au réacteur de biogaz en cas de passage sous la valeur indicative par la concentration déterminée en éléments traces,
- les valeurs indicatives sont de 4 à 30 mg/kg de matière sèche pour le nickel et/ou de 0,4 à 5 mg/kg de matière sèche pour le cobalt et/ou de 1,0 à 10,0 mg/kg de matière sèche pour le molybdène et/ou de 1500 à 3500 mg/kg de matière sèche pour le fer.

**2.** Procédé selon la revendication 1, dans lequel des valeurs indicatives sont fournies pour la concentration des éléments traces nickel et/ou cobalt et/ou molybdène et/ou fer, et la concentration des éléments traces nickel et/ou cobalt et/ou molybdène et/ou fer dans le réacteur de biogaz est déterminée.

**3.** Procédé selon une des revendications 1 ou 2, dans lequel les valeurs indicatives sont d'au moins 10 et/ou au maximum de 25 mg/kg de matière sèche pour le nickel et/ou d'au moins 1,0 mg/kg de matière sèche pour le cobalt.

**4.** Procédé selon une des revendications 1 à 3, dans lequel des valeurs indicatives sont fournies pour les concentrations des éléments traces manganèse et/ou cuivre et/ou sélénium et/ou tungstène et/ou zinc, et dans lequel les concentrations des éléments traces manganèse et/ou cuivre et/ou sélénium et/ou tungstène et/ou zinc dans le réacteur de biogaz sont déterminées.

**5.** Procédé selon la revendication 4, dans lequel les valeurs indicatives sont de 100 à 1500 mg/kg de matière sèche pour le manganèse et/ou de 10 à 80 mg/kg de matière sèche pour le cuivre et/ou de 0,05 à 4 mg/kg de matière sèche pour le sélénium et/ou de 0,1 à 30 mg/kg de matière sèche pour le tungstène et/ou de 30 à 400 mg/kg de matière sèche pour le zinc.

**6.** Procédé selon la revendication 5, dans lequel les valeurs indicatives sont d'au moins 250 et/ou au maximum de 350 mg/kg de matière sèche pour le manganèse et/ou d'au moins 30 et/ou au maximum de 50 mg/kg de matière sèche pour le cuivre et/ou d'au moins 0,3 et/ou au maximum de 0,7 mg/kg de matière sèche pour le sélénium et/ou d'au moins 0,4 et/ou au maximum de 0,8 mg/kg de matière sèche pour le tungstène et/ou d'au moins 150 et/ou au maximum de 250 mg/kg de matière sèche pour le zinc.

**7.** Procédé selon une des revendications 1 à 6, dans lequel la disponibilité biologique des éléments traces contenus dans le biomatériau dans le réacteur de biogaz est augmentée.

8.  Procédé selon la revendication 7, dans lequel une addition augmentant la disponibilité biologique des éléments traces est conduite au réacteur de biogaz.

9.  Procédé selon la revendication 8, dans lequel l'addition contient du fer.

10. Procédé selon une des revendications 7 à 9, dans lequel au moins un élément trace est ajouté après l'augmentation de la disponibilité biologique des éléments traces.

11. Procédé selon une des revendications 7 à 10, dans lequel, après la reprise de la disponibilité biologique des éléments traces, la concentration d'au moins un élément trace dans le biomatériau est déterminée, et un manque de l'élément trace est compensé par l'ajout de ce même élément.

12. Procédé selon une des revendications 1 à 11, dans lequel la concentration d'au moins un élément trace dans au moins un échantillon provenant du réacteur de biogaz est déterminée par analyse ICP.

13. Procédé selon une des revendications 1 à 12, dans lequel les concentrations d'au moins un élément trace dans le réacteur de biogaz sont déterminées de façon répétée à intervalles de temps.

14. Procédé selon une des revendications 1 à 13, dans lequel la quantité à ajouter des éléments traces est déterminée en fonction de la différence de la valeur indicative et de la concentration déterminée.

15. Procédé selon la revendication 14, dans lequel la quantité à ajouter des éléments traces est déterminée en prenant en compte les éléments traces prélevés dans le réacteur de biogaz avec les résidus de fermentation.

16. Procédé selon une des revendications 1 à 15, dans lequel, initialement, seule une partie de la quantité à ajouter d'éléments traces est ajoutée, et puis plus tard des quantités correspondant au besoin en éléments traces à ajouter sont ajoutées.

17. Procédé selon la revendication 16, dans lequel, initialement, à l'intérieur d'une période d'une à deux semaines, une partie de la quantité à ajouter d'éléments traces est ajoutée.

18. Procédé selon une des revendications 1 à 17, dans lequel les éléments traces sont ajoutés de façon continue ou unique ou répétée et/ou sont ajoutés par un ajout unique ou répété d'un dépôt qui libère des éléments traces sur une période plus longue.

19. Procédé selon une des revendications 1 à 18, dans lequel une addition comprenant différents éléments traces est ajoutée au réacteur de biogaz.

20. Procédé selon la revendication 19, dans lequel l'addition est réalisée spécialement en fonction des valeurs indicatives et des concentrations déterminées.

21. Procédé selon la revendication 19, dans lequel plusieurs ajouts comprenant différents éléments traces sont réalisés avec différents rapports quantitatifs des éléments traces et dans lequel, parmi ces additions, c'est l'addition dont la composition se rapproche le plus de la composition, déterminée à l'aide des valeurs indicatives et des concentrations déterminées, de l'adition à ajouter au réacteur de biogaz qui est conduite au réacteur de biogaz.

## Fos/Tac-Entwicklung und Energieertrag beim Einsatz einer Mineralstoffmischung

EP 1 997 901 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1577269 A1
- SE 529177 C2

**In der Beschreibung aufgeführte Nicht-Patentliteratur**

- SAHM: Biologie der Methanbildung. *Chem.-Ing. Tech.,* 1981, vol. 53 (11), 854-863
- **ARESTA, M. et al.** INFLUENCE OF IRON; NICKEL AND COBALT ON BIOGAS PRODUCTION DURING THE ANAEROBIC FERMENTATION OF FRESH RESIDUAL BIOMASS. *CHEMISTRY AND ECOLOGY,* 2003, vol. 19 (6), 451-459
- **WILKY, A. et al.** Enhancement of Ananerobic Methanogenesis from Napiergrass by Addition of Micronutrients. *BIOMASS,* 1986, vol. 11, 135-146